**Europäisches Patentamt**

**European Patent Office**

**Office européen des brevets**

(19)

(11) Veröffentlichungsnummer: **0 052 328**
**B1**

(12) **EUROPÄISCHE PATENTSCHRIFT**

(45) Veröffentlichungstag der Patentschrift:
**16.01.85**

(51) Int. Cl.⁴: **G 01 N 33/18, G 01 N 31/22,**
**G 01 N 33/52**

(21) Anmeldenummer: **81109588.4**

(22) Anmeldetag: **10.11.81**

(54) **Analytisches Mittel.**

(30) Priorität: **19.11.80 DE 3043608**

(43) Veröffentlichungstag der Anmeldung:
**26.05.82 Patentblatt 82/21**

(45) Bekanntmachung des Hinweises auf die Patenterteilung:
**16.01.85 Patentblatt 85/3**

(84) Benannte Vertragsstaaten:
**AT BE CH DE FR GB IT LI LU NL SE**

(56) Entgegenhaltungen:
**DE - A - 1 598 224**
**DE - A - 2 015 271**
**DE - A - 2 711 201**
**DE - A - 2 739 008**
**US - A - 3 595 754**

(73) Patentinhaber: **BEHRINGWERKE**
**AKTIENGESELLSCHAFT, Postfach 1140,**
**D-3550 Marburg/Lahn (DE)**

(72) Erfinder: **Habenstein, Klaus, Dr., Im Ketzergrund 33,**
**D-3552 Wetter (DE)**
Erfinder: **Kohl, Helmut, Dr., Goethestrasse 32,**
**D-3552 Wetter (DE)**
Erfinder: **Walch, Axel, Dr., Hans-Sachs-Strasse 5,**
**D-6000 Frankfurt am Main (DE)**

(74) Vertreter: **Meyer-Dulheuer, Karl-Hermann, Dr. et al,**
**HOECHST Aktiengesellschaft Zentrale Patentabteilung**
**Postfach 80 03 20, D-6230 Frankfurt/Main 80 (DE)**

ACTORUM AG

## Beschreibung

Die Erfindung betrifft ein analytisches Mittel zum Nachweis von Inhaltsstoffen von wässrigen oder wasserenthaltenden Flüssigkeiten mit chromatographischen Methoden.

Bei analytischen Screeningverfahren ist seit Jahren ein starker Trend zum Einsatz von Schnelltests auf der Basis von Teststäbchen zu beobachten, worunter im wesentlichen flächige Gebilde wie Teststreifen verstanden werden. Es sind jedoch auch folienumhüllte Teststreifen oder röhrchenförmige Gebilde bekannt (DE-AS 14 98 875, DE-OS 19 40 964). Die Anwendung dieser Schnelltests erfolgt entweder durch Eintauchen eines mit den testnotwendigen Chemikalien getränkten saugfähigen Materials, z. B. Papier, in die zu untersuchende Probe und Beobachtung einer auftretenden Farbreaktion oder Eintauchen eines Teils des chemikaliengetränkten Papiers, chromatographisches Aufziehen der Probe und Beobachtung einer Verfärbung im nichtgetauchten Teil des Papiers.

Chromatographische Schnelltests der letztgenannten Art werden im allgemeinen eingesetzt, wenn vor, während oder nach der Reaktion der testnotwendigen Chemikalien mit der nachzuweisenden Substanz eine Abtrennung der nachzuweisenden Substanz von anderen Probeinhaltsstoffen, der Reaktionsprodukte von den testnotwendigen Chemikalien oder verschiedener Reagenzien voneinander erreicht werden soll.

Wesentliche Voraussetzung für ein einwandfreies, reproduzierbares Funktionieren chromatographischer Schnelltests ist eine gleichmässige chromatographische Durchfeuchtung des nicht eingetauchten Teils des saugfähigen Materials, was seinerseits über ein gleichbleibendes und reproduzierbares Flüssigkeitsangebot an einer genau definierten Startlinie des chromatographisch wirksamen Materials erreicht wird.

Aus den US-Patentschriften 2, 785, 057 und 3, 915, 647 ist ein Schnelltest bekannt, der aus einem chromatographisch wirksamen Material und einem fest damit verbundenen Probengefäss besteht, wobei das Probengefäss über flüssigkeitsdurchlässige Öffnungen mit dem saugfähigen Material in Kontakt steht.

Die Herstellung dieses Schnelltests ist teuer, und der Teststreifen muss nach dem Tauchen stets senkrecht gehalten werden. Weiterhin sinkt durch Einsaugen der Flüssigkeit in das chromatographisch wirksame Material der Flüssigkeitsspiegel im Reservoir und damit verändert sich die Startlinie der Chromatographie während des Testes laufend, was zu erheblichen Schwankungen der Chromatographiedauer führt.

Nach der DE-OS 22 15 089 wird das chromatographisch aktive Material mit einer Schicht eines quellfähigen Gebildners verbunden, die als Flüssigkeitsreservoir dient.

Dieses Verfahren führt zu einem im feuchten Zustand mechanisch sehr instabilen, leicht verschmierenden Produkt, wobei das Gel zudem die Feuchtigkeit selbst stark festhält und der Quellvorgang langsam ist.

Von diesen analytischen Mitteln erfüllt keines die an einen chromatographischen Schnelltest zu stellenden Anforderungen: Einfache Handhabung, problemlose und kostengünstige Herstellung sowie reproduzierbare Chromatographieeigenschaften (Laufhöhe, Laufzeit) durch gleichmässiges Flüssigkeitsangebot.

Der Erfindung lag somit die Aufgabe zugrunde, diese Anforderungen in einem verbesserten chromatographischen Schnelltest, bestehend aus chromatographisch aktivem Material und einem Flüssigkeitsreservoir mit reproduzierbarer Flüssigkeitsaufnahme zu vereinen.

Überraschend wurde nun gefunden, dass poröse hydrophile, nicht gelbildende, quellfähige Polymere sich selbst bei ungleichmässiger und grober Porenstruktur ausgezeichnet als selbstansaugende Flüssigkeitsreservoire mit sehr hoher und gleichmässiger Flüssigkeitsaufnahme und -abgabe in einem chromatographischen Schnelltest eignen.

Die Herstellung poröser hydrophiler, quellfähiger Polymerer ist z. B. in DE-OS 27 39 008 und DE-OS 27 22 025, Seiten 8 und 9, beschrieben.

Geeignete Polymersubstanzen hydrophiler Natur sind demnach beispielsweise regenerierte und modifizierte Cellulosetypen, gegebenenfalls chemisch vernetzt, wasserunlösliche Pfropfpolymerisate aus Cellulosen und synthetischen hydrophilen Polymerbildern wie Polyacrylamiden, Polyacrylsäuren, Polyglykolen und Polyvinylpyrrolidonen sowie diese synthetischen Polymerbildner allein in vernetzter Form.

Bevorzugt sind regenerierte, modifizierte und/oder vernetzte Cellulosetypen.

Als sehr vorteilhaft im Sinne der Erfindung erweist sich die erhebliche Quellfähigkeit in wässrigen Medien. Ein trockner Schwamm aus diesen Polymeren kann beispielsweise durch einen einfachen Pressvorgang auf $\frac{1}{10}$ des Nassvolumens verpresst werden und behält diese Form bis zur Anfeuchtung bei. Dabei können sowohl flächige, papierähnliche Strukturen als auch Pulver, Flocken oder andere Formkörper verwendet werden. Für die Herstellung von Teststreifen wird man zweckmässigerweise ein trockengepresstes flächiges Material von der gleichen Trockenstärke wie das verwendete chromatographisch aktive Material einsetzten, da dann mit geringsten produktionstechnischen Schwierigkeiten zu rechnen ist.

Als chromatographisch aktives Material sind Papiere aus Cellulose oder Cellulosederivaten sowie stabilisierte für eine Chromatographie geeignete Beschichtungen aus Kieselgelen und dergleichen brauchbar.

Die folgenden Beispiele sollen die Erfindung erläutern:

Beispiel 1: Teststreifen zur Bestimmung von alpha-Amylase im Urin

Auf eine Kunststoff-Folie von 0,3 mm Dicke, 7 mm Breite und 10 cm Länge wird an einem Ende ein 10 mm langes Stück trocken-gepresster Kunstschwamm aus regenerierter Cellulose von 7 mm Breite und 0,2 mm Dicke aufgeklebt. Daran angrenzend wird ein Stück Filterpapier von 40 mm Länge, 7 mm Breite und 0,2 mm Dicke aufgeklebt. Auf dem Papier sind ein oder mehrere Striche einer wasserunlöslichen chromogenen Stärkeverbindung quer zur Teststreifenrichtung aufgetragen.

Zur Ausführung des Testes wird das untere Drittel des mit dem Kunstschwamm beklebten Teststreifenendes in Urin eingetaucht. Unter Ausdehung auf etwa die 10-fache Dicke werden von dem Kunstschwamm 120 µl Urin aufgesaugt und zur Stosskante mit dem Filterpapier weitertransportiert. Dort beginnt nach ca. 2 Sekunden der Chromatographiervorgang, der nach ca. 5 Minuten beendet ist. Der Amylasegehalt der Probe lässt sich an der Zahl der während dieser 5 Minuten abgelösten Stärkestriche halbquantitativ ablesen.

In anderen Ausführungsformen kann ein Schwamm selbst bereits Chemikalien oder andere testnotwendige Substanzen (z. B. Enzyme) enthalten, wobei das Flüssigkeitsaufnahmevermögen geringfügig abnimmt.

Eine weitere technisch nutzbare Eigenschaft des Kunstschwamms ergibt sich daraus, dass der gepresste Schwamm nur mit Wasser quellbar ist und somit auch in stark organisch verunreinigten Abwässern optimal angewandt werden kann, ohe dass das Flüssigkeitsaufnahmevermögen bereits durch nichtwässrige Lösungsmittel abgesättigt wird.

Beispiel 2: Test auf Eisengehalt im Abwasser (siehe Zeichnung)

Ein Plastikröhrchen (1) (Makrolon$^{(R)}$) mit 6 mm Innendurchmesser wird an einem Ende mit einer wasserdurchlässigen Lochplatte (2) verschlossen, daraufhin werden ca. 1 ml einer lockeren Schüttung eines Pulvers aus regenerierter Cellulose mit einer Teilchengrösse von ca. 1 – 2 mm Durchmesser (3) eingefüllt und mit Hilfe eines nachgeschobenen Papierstopfens (4) auf 1 ml (= 3 cm Röhrchenlänge) verdichtet. Darüber wird eine mit Kaliumhexacyanoferrat (II) imprägnierte Papierhülse (5) mit einem Aussendurchmesser von 6 mm angeordnet, die mit Hilfe eines Abschlussstopfens (6) gegen den Papierstopfen (4) gepresst wird.

Zur Ausführung des Tests wird das Röhrchen mit der wasserdurchlässigen Lochplatte ca. 6 Sekunden in die Abwasserprobe getaucht. Die in dieser Zeit aufgenommene definierte Flüssigkeitsmenge wandert in die Papierhülse (5), wo mit Fe(III)-Salzen das schwer lösliche Berlinerblau entsteht. Nach Beendigung der Chromatographie ist die Fe(III)-Salzkonzentration in der Abwasserprobe über die Höhe der Berlinerblau-Zone in der Papierhülse (5) anhand einer Eichkurve abzulesen.

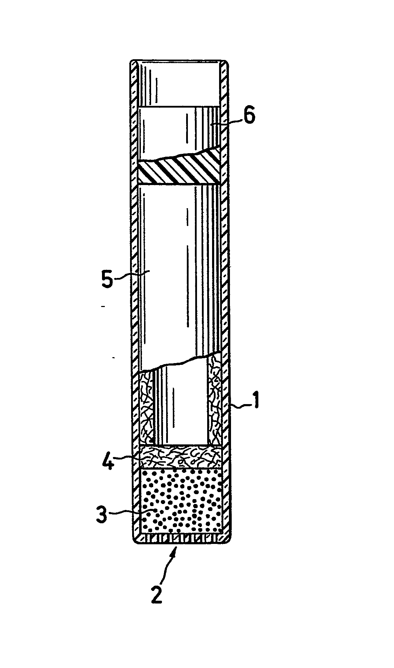

**Patentansprüche**

1. Analytisches Mittel zum Nachweis von Inhaltsstoffen von wässrigen oder wasserenthaltenden Flüssigkeiten mit chromatographischen Methoden, bestehend aus mindestens zwei in Kontakt miteinander befindlichen flüssigkeitsaufnehmenden Zonen, wovon mindestens eine aus einem chromatographisch aktiven Material besteht und eine andere als Flüssigkeitsreservoir dient, sowie einem diese Anordnung stabilisierenden, flächigen oder hohlkörperförmigen Träger, dadurch gekennzeichnet, dass die als Flüssigkeitsreservoir dienende Zone aus einem hydrophilen quellfähigen Polymeren besteht, das gegebenenfalls Chemikalien oder andere testnotwendige Substanzen enthalten kann.

2. Analytisches Mittel gemäss Anspruch 1, dadurch gekennzeichnet, dass das Flüssigkeitsreservoir ein Kunststoffschwamm aus einem hydrophilen quellfähigen Polymeren in beliebiger, dem jeweiligen Test optimal angepasster Form und Grösse ist.

3. Analytisches Mittel gemäss Anspruch 1, dadurch gekennzeichnet, dass das hydrophile quellfähige Polymer aus regenerierter Cellulose oder einem Cellulosederivat besteht.

**Claims**

1. An analytical means for the detection of ingredients of aqueous or water-containing fluids by chromatographic methods, comprising at least two fluid-absorbing zones which are in mutual contact and of which at least one is composed of a chromatographically active material and another serves as a fluid reservoir, and a carrier which is two-dimensional or has the form of a hollow body and stabilizes this arrangement, wherein the zone serving as a fluid reservoir is composed of a hydrophilic swellable polymer which, if appropriate, can contain chemicals or other substances necessary for the test.

2. An analytical means as claimed in Claim 1, wherein the fluid reservoir is a plastic sponge of a hydrophilic swellable polymer in any desired form and size, adapted in an optimum manner to the particular test.

3. An analytical means as claimed in Claim 1, wherein the hydrophilic swellable polymer is composed of regenerated cellulose or a cellulose derivative.

**Revendications**

1. Dispositif analytique pour la détection de constituants de liquides aqueux ou contenant de l'eau par des méthodes chromatographiques, se composant d'au moins deux zones fixant du liquide se trouvant en contact l'une avec l'autre, dont l'une au moins se compose d'une matière chromatographiquement active et l'autre sert de réservoir de liquide, ainsi que d'un support plat ou en forme de corps creux, stabilisant ce dispositif, caractérisé en ce que la zone servant de réservoir de liquide se compose d'un polymère hydrophile capable de gonfler, qui peut le cas échéant contenir des produits chimiques ou d'autres substances nécessaires à l'essai.

2. Dispositif analytique suivant la revendication 1, caractérisé en ce que le réservoir de liquide est une éponge artificielle en un polymère hydrophile capable de gonfler, dans n'importe quelle forme et dimension adaptée de manière optimale à chaque essai.

3. Dispositif analytique suivant la revendication 1, caractérisé en ce que le polymère hydrophile capable de gonfler se compose de cellulose régénérée ou d'un dérivé de la cellulose.